# EUROPEAN PATENT APPLICATION

(11) **EP 0 791 328 A1**
(43) Date of publication of application: **27.08.1997**
(21) Application number: 97200488.1
(22) Date of filing: 20.02.1997
(51) Int. Cl.: A61B 5/11

(54) **Measuring the motor activity of a subjet**

(30) Priority: 21.02.1996 NL 1002412
(71) Applicant: Van Lummel, Robert Christiaan, 2596 HN Den Haag (NL)
(72) Inventor: Van Lummel, Robert Christiaan, 2596 HN Den Haag (NL)
(74) Representative: Smulders, Theodorus A.H.J., Ir.

(57) **Abstract**

A device for measuring the gross motor activity of a subject, for instance a human comprises at least one motion sensor (2) worn as a belt (1) and fastened to the subject. The measurement data of the sensor (2) are transferred to a suitably programmed calculating device which converts those data to data regarding position and/or motion and/or motional change of the being. A sensor system with at most three sensors arranged in mutual proximity is used. If more than one sensor is used, the sensors are directed orthogonally with respect to each other. The sensor system is fastened at such a point and in such a manner that the position or motional change of the pelvis of the subject is measured.

## Description

The invention relates to a method for measuring the gross motor activity of a living being, in which at least one motion sensor is fastened on or to the body of that being and the measurement data of the at least one sensor are transferred to a suitably programmed calculating device which converts those data to data regarding position and/or motion and/or motional change of the being. The invention further relates to a sensor device for use in such a method.

Gross motor activity is commonly understood to mean a motor activity in which the body in its entirety is involved in some manner or other. As main categories of gross motor activity, the following can be mentioned: sitting, standing, lying, locomotion (going from one place to another) and moving the body at or adjacent a particular point (for instance, swinging). Gross motor activity can be regarded in contrast with fine motor activity, such as, for instance, writing.

A method as mentioned in the opening paragraph hereof is known, for instance, from an article in Klinische Fysica, 1995/4, pp. 9-13. A sensor device for registration of motions or positions of the body of, for instance, a human is described in PCT application WO 81/01506.

In the known method, it is conventional to measure with the aid of sensors attached here and there to the subject's body. As a consequence, the subject's freedom of movement is limited. In the known method, however, it is necessary to attach the sensors at points distributed over the body because the method requires measurement on a number of different segments of the body to be able to furnish the desired information regarding position and/or motion.

Now, one object of the invention is to provide a method whereby the freedom of movement of the individual to be examined (the living being) is limited to a minimum extent, while nevertheless the posture of the body and motion can be determined with a high degree of accuracy. Another object of the invention is to provide a sensor device which can be suitably used in that method.

The stated object is realized through a method in which a sensor system with at most three sensors arranged in mutual proximity is used, and if more than one sensor is used, the sensors are directed orthogonally with respect to each other, which sensor system is attached to the being at such a point and in such a manner that the position or motional change of the pelvis of the being is measured. In a suitable embodiment of the method according to the invention, a single sensor is used.

The sensor device according to the invention is characterized by a sensor system with at most three sensors arranged in mutual proximity, while if more than one sensor is provided, those sensors are directed in mutually orthogonal directions, which sensor system is arranged in a girdle adapted to be worn as a belt or a strap by the living being to be examined.

It is noted that U.S. Patent 4,108,164 discloses a device with which the position of an individual's spine and the extent of bending (for instance when pumping a bicycle tyre) can be determined. The device is in the form of a jacket or harness with sensors at three points along the spine and with leads running along the sleeve of the jacket for connecting the sensors with gauges to be attached to the subject's wrist. With such a device it is not possible to measure the general gross motor activity, and further the freedom of movement of the individual who uses the device does not seem optimal.

Further, U.S. Patent 5,146,929 discloses a portable instrument for measuring movement of a subject's spine, consisting of at least two mutually coupled boxes or housings to be fastened to the subject's spine, and a plate to be fastened to the subject's spine at a distance from those boxes. The boxes are fastened around the subject's waist by means of a belt, and the plate is fastened around the subject's chest by means of a second belt or strap. Not only does it seem impossible with this device to measure the position or motional change of the subject's pelvis, it also seems the freedom of movement of the subject is not optimal.

In a suitable embodiment of the sensor device according to the invention, the sensor system comprises two sensors, of which, upon attachment of the girdle with the sensor system to the living being, one is directed along the body axis of the living being and the other in a direction perpendicular to that body axis, corresponding to the direction of normal locomotion of the living being.

According to the invention, therefore, measurement is no longer performed on different body segments, but involves only the position, movement and/or motional change of the subject's pelvis. Surprisingly, it has been found that, using a calculating device, from measurements of those quantities the subject's gross motor activity can be derived. The software by means of which this can be done is known to the skilled artisan - at any rate, he will be able to design such software in a known manner - and does not form part of the present invention. By virtue of the fact that only one sensor system according to the invention needs to be used, which is attached at a position not unusual for the subject (the girdle), the limitation of the subject's freedom of movement is minimal.

The invention is elucidated with reference to the drawings, in which:
Fig. 1 is a diagrammatic representation of an embodiment of the sensor device according to the invention, worn by a subject, and
Fig. 2 is a representation in side elevation of subjects with different gross motor activities.

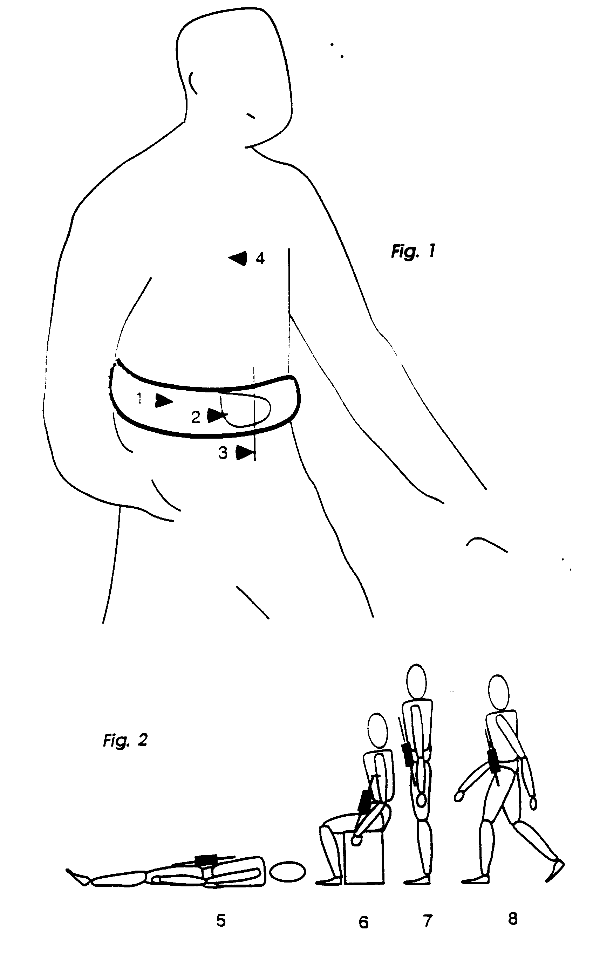

In Fig. 1 a subject 4 is represented schematically. The subject 4 wears a girdle or strap 1, in which a sensor system 2 is accommodated. The strap 1 consists of a suitable material, for instance a plastic material. The subject 4 has the girdle 1 fastened around the waist, that is, at the level of the pelvis of the subject 4. The software for determining the gross motor activity of the subject 4 on the basis of measurement by the sensor system preferably provides an adjustment for the starting position of the body axis 3 in the standing position of the subject. This starting position is determined before further measurements on position and/or motional changes are performed.

It has been found that the position of the pelvis of a subject is different for the various gross motor activities, so that measuring that position provides information about the gross motor activity.

In Fig. 2 a number of different gross motor activities are represented diagrammatically. The lying posture is designated at 5, the subject 6 is sitting, the standing posture is represented by the subject 7, and locomotion (walking) is indicated at 8.

The reason why the position or motional change of the waist (the pelvis) can be translated to gross motor activity is the following. The position of the subject's pelvis is different for the postures of lying 5, sitting 6 and standing 7. It moreover appears that the motional change of the girdle with the sensor system is different for locomotion (walking) and arbitrary motion. Further, the frequency of the locomotion can be determined.

Use of the maximum number of three sensors in the sensor system allows every gross motor activity to be determined and measured, because it affords the possibility of measuring in three mutually orthogonal directions. If there is an interest in only one gross motor activity, fewer sensors can suffice. For measuring locomotion (walking) a single sensor directed along the subject's body axis is already sufficient.

## Claims

1. A method for measuring the gross motor activity of a living being, in which at least one motion sensor is fastened on or to the body of that being and the measurement data of the at least one sensor are transferred to a suitably programmed calculating device which converts those data to data regarding position and/or motion and/or motional change of the being,
characterized in that a sensor system with at most three sensors arranged in mutual proximity is used, and if more than one sensor is used, the sensors are directed orthogonally with respect to each other, which sensor system is fastened to the being at such a point and in such a manner that the position or motional change of the pelvis of the being is measured.

2. A method according to claim 1, characterized in that a sensor system with a single sensor is used.

3. A sensor device for use in the method according to claim 1, characterized by a sensor system with at most three sensors arranged in mutual proximity, while if more than one sensor is provided, those sensors are directed in mutually orthogonal directions, which sensor system is arranged in a girdle adapted to be worn as a belt or a strap by the living being to be examined.

4. A sensor device according to claim 3, characterized in that the sensor system contains two sensors, of which, upon attachment of the girdle with the sensor system to the living being, one is directed along the body axis of the living being and the other in a direction perpendicular to that body axis, corresponding to the direction of normal locomotion of the living being.

5. A sensor device according to claim 3, characterized in that the sensor system contains a single sensor.
